# EUROPEAN PATENT SPECIFICATION

(11) **EP 1 132 090 B1**
(45) Date of publication and mention of the grant of the patent: **13.08.2003**
(21) Application number: 99958545.8
(22) Date of filing: 16.11.1999
(51) Int. Cl.: A61K 35/78

(54) **METHOD FOR PRODUCING AN AGENT FOR TREATING ULCERS OF THE ORGANISM**
METHODE ZUR HERSTELLUNG EINES WIRKSTOFFES ZUR BEHANDLUNG VON ULCERA DES ORGANISMUS
PROCEDE DE PRODUCTION D'UN AGENT PERMETTANT DE TRAITER DES ULCERES DE L'ORGANISME

(30) Priority: 16.11.1998 RU 98120572; 01.11.1999 RU 99122586
(43) Date of publication of application: 12.09.2001
(73) Proprietor: Obschestvo & Organichennoi Otvetstvennostiju Ligpharm, Moscow, 103051 (RU)
(72) Inventor: PETUKHOV, Dmitry Viktorovich, St.Petersburg, 198004 (RU); TROFIMOV, Valery Aphanasievich, St.Petersburg, 194156 (RU); FILOV, Vladimir Alexandrovich, St.Petersburg, 197871 (RU); REZTSOVA, Valentina Vasilievna, St.Petersburg, 189640 (RU); KILMAEVA, Nadezhda Egorovna, Leningradskaya obl., 188650 (RU); MARKELOV, Oleg Jurievich, St.Petersburg, 196135 (RU); PINCHUK, Boris Timopheevich, St.Petersburg, 198004 (RU); SMIRNOV, Jury Alexeevich, St.Petersburg, 196158 (RU)
(74) Representative: W.P. Thompson & Co.
(86) International application number: RU9900439
(87) International publication number: WO00029005

(56) References cited:
- EP-A- 1 142 578
- EP-A1- 0 595 297
- RU-C1- 2 084 236
- RU-C1- 2 094 053
- US-A- 5 510 337
- DATABASE WPI Section Ch, Week 9838 Derwent Publications Ltd., London, GB; Class B04, AN 98-444852 XP002183913 & RU 2 102 083 C (PETUKHOV DV), 20 January 1998 (1998-01-20)

## Description

### TECHNICAL FIELD OF THE INVENTION

The present invention relates to the process for producing the medicinal preparations.

### PRIOR ART

The method has been previously proposed for the production of the remedy for treating non-oncological pathologies of organism which comprises the treatment of lignin-containing raw material by isolating the soluble substances with the obtainment of the preparation 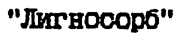 (Lignosorbum). This preparation is a paste containing lignin and up to 20% residual polysaccharides and it is used as a non-specific entero-sorbent (BΦC 42-2203-93).

The drawback of this method lies in obtaining the remedy of the narrow range of application.

### DISCLOSURE OF THE INVENTION

The technical result of the invention is the production of the remedy with the widened range of application.

This result is achieved in the following way. In the method for producing the remedy for treating ulcers involving the treatment of lignin-containing raw material, according to the invention the raw material is subjected to the basic hydrolysis-extraction and the treated material is oxidized with the oxygen-containing gas; liquid phase is separated and acidified and the desired precipitated product is separated.

This allows to obtain the remedy for treating ulcers.

In a preferred embodiment of the present invention the procedure of hydrolysis-extraction is realized in the field of ultrasonic oscillations, preferably under the conditions of the developed cavitation.

This gives the possibility to intensify the process and to increase the capacity of the method.

In another preferred embodiment of the invention the carrier is introduced into the product obtained.

This allows to produce the remedy in various preparative forms, which facilitates its use when treating the pathologies of different localizations.

Still another preferred embodiment of the present invention envisages the steps of: washing the precipitate till neutral pH is reached, drying the precipitate, mixing the precipitate with pyrophosphate at a dry mass ratio of (25:17)±10%, dissolving the mixture in water while heating and adding NaCl to the mixture in order to obtain the desired product.

Such a remedy shows a greater efficiency.

In this case it is possible to add HCl into the product to adjust pH to a neutral value.

The injections of such remedy are less painful.

### BEST MODE FOR CARRYING OUT THE INVENTION

The method is realized in the following way.

The lignin-containing raw material is made free of the soluble components and it is subjected to the basic hydrolysis-extraction, preferably in the field of ultrasonic oscillations and the most preferably under the conditions of the developed cavitation, then it is oxidized with the oxygen-containing gas and the reaction mixture is cooled and filtered. The separated liquid phase is acidified until formation of the precipitate, which is separated from the liquid phase. After washing and drying the precipitate is a water-insoluble dark-brown powder without taste and odour, comprising polyoxycarboxylic acids. Then, in a preferred embodiment, the powder is blended with the salt of pyrophosphoric acid at a dry mass ratio of 25:17 accurate up to 10% in terms of pyrophosphate ion; the blend is dissolved in water while heating, for example in a water bath, and NaCl is added to the blend in the amount of 3 mass parts accurate up to 10% in the form of the dry matter or the physiological salt solution and it is desirable to add HCl until reaching neutral pH within the same accuracy. Introduction of the produced remedy into organism causes weakening the reaction of lipids peroxidation in the tissues which is intensified, as is generally known, under any excessive actions of the factors of psychical, physical or chemical nature on the organism. Activation of lipids peroxidation is of great importance in the development of atherosclerosis, ischemia, myocardial infarction, tuberculosis and inflammatory processes. Intensification of lipids peroxidation in the stomach mucosa results in necrosis and ulcer formation. Accumulation of lipids peroxidation products in the tissues is accompanied by the disturbance of the functions of some enzymatic systems. On the basis of the above-mentioned the conclusion is drawn that the remedy obtained according to the proposed method is effective for treating ulcers because it promotes the decrease of the activity of lipids peroxidation.

The remedy obtained may be used in various preparative forms for the treatment of the pathologies of different localizations. Thus, for the external use it is expedient to make an unguent by introducing the obtained product, for example, into glycerine; for rectal use it is possible to make suppositories containing the obtained product, for example on cacao butter base, and for injections the product may be dissolved, for example in 0.2 M tetrasubstituted sodium pyrophosphate, and diluted, for example with the physiological salt solution.

### EXAMPLE 1

The remedy is produced by hydrolysis-extraction of the medicinal lignin in sodium hydroxide at the phases ratio of 1:10 and at a temperature of 70°C for 20 hours, oxidizing the treated material with air at 190°C for 2.5 hours, filtering the material, acidifying liquid phase and separating the formed precipitate with subsequent washing and drying.

### EXAMPLE 2

The remedy is produced by hydrolysis-extraction of the lignin-containing preparation 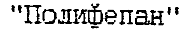 (Polyphepan) in potassium hydroxide at the cavitation index of 1.4, the phases ratio of 1:8 and a temperature of 80°C for 25 minutes. Then the treated preparation is oxidized with oxygen at 200°C for 30 minutes and filtered; the liquid phase is acidified and the precipitate is separated with subsequent washing and drying.

### EXAMPLE 3

The remedy is obtained by hydrolysis-extraction of the medicinal lignin in sodium hydroxide at the phases ratio of 1:10 and a temperature of 70°C for 20 hours and subsequent oxidizing the treated material with air at a temperature of 190°C for 2.5 hours. Then the material is filtered and the liquid phase is acidified. The formed precipitate is separated with subsequent washing till neutral pH is reached, drying, mixing with crystalline hydrate of sodium pyrophosphate (Na₄P₂O₇·10H₂O) according to ΓOCT 342-77, in a ratio mentioned above and dissolving in water while heating. Then NaCl is introduced into the product in the predetermined amount accurate up to ±10% as provided by the used dispensers.

### EXAMPLE 4

The remedy is produced in a similar manner to that of Example 3 with introduction of HCl at the final stage till neutral pH value is reached.

The properties of the remedy obtained according to the pair of examples 1 and 2 did not differ from the properties of the remedy obtained according to the pair of examples 3 and 4.

In order to verify the attainment of the technical result of the present invention there were made the experiments on determination of the application efficiency of the remedy named 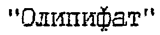 (Olipyphate) in cases of the different pathologies.

The experiments on treating chronic ulcerous disease of the stomach were carried out on the mongrel gray rats according to the Vertelkin's method using cryogenic action on the stomach for developing ulcers. For the purpose of comparison of the therapeutic effect of Olipyphate and the traditionally used preparations for treating the ulcerous disease the bloc-kader of H2-histamine receptors 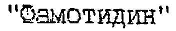 (Famotidine) was administered perorally to male rats daily for 10 days in the dose of 40 mg/kg and reparant 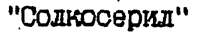 (Solcoseryl) was injected to male rats daily for 10 days in the dose of 0.5ml/kg. The results of the action of the preparations are given in Table 1 and Table 2, where the figures in the round brackets correspond to the numbers of the examples of Olipyphate production.

Injections of the remedy obtained according to the Example 4 were less painful as it was evaluated by the development of edema and indirectly by the pulse and breathing frequency increase and normalization.

The given data show that the action of Olipyphate is to some extent weaker than the action of the traditionally used preparations and efficiency does not depend on the sex of animals. Three-fold intramuscular injections at a dose of 10 mg/kg are the most effective.

When carrying out the experiments in vitro there was determined the efficiency of the remedy used for treating surface ulcers, including the trophic ones.

Thus, the proposed method allows to obtain the remedy of wide range of application for the treatment of ulcers.

| Change of the cryogenic uloer area and malonic dialdehyde content in the stomach tissue of rats under the action of the separations used | | |
|---|---|---|
| Run of the experiments | Ulcer area, mm² | MDA, nM/g of tissue |
| Intact animals | 0 | 7.47 ± 0.96 |
| Control rats | 18.50 ± 2.50 | 11.23 ± 1.42 |
| Solcoseryl, 10 times, daily, i.m. | 10.32 ± 2.90 | 8.99 ± 0.74 |
| Famotidine, 10 times, daily, per os | 13,43 ± 2.33 | 9.54 ± 0.71 |
| Olipyphate (1;2), 50 mg/kg, 5 times daily, i.m. | 11.90 ± 3.55 | 9.95 ± 0.63 |
| Olipyphate (1;2), 50 mg/kg, 5 times every second day, i.m. | 12.97 ± 2.05 | 9.63 ± 0.39 |
| Olipyphate (1;2), 100 mg/kg, 5 times daily, i.m. | 13.75 ± 2.53 | - |
| Olipyphate (1;2), 100 mg/kg, 5 times daily, per rectum, suppositories | 13.37 ± 1.65 | - |
| Olipyphate (1;2), 50 mg/kg, 5 times daily, per os | 22.00 ± 3.39 | - |
| Olipyphate (1;2), 100 mg/kg, 5 times daily, per os | 18.90 ± 3.12 | - |
| Olipyphate (3;4), 50 mg/kg, 5 times daily, i.m. | 11.66 ± 3.05 | 9.65 ± 0.63 |
| Olipyphate (3;4), 50 mg/kg, 5 times every second day, i.m. | 12.37 ± 2.05 | 9.59 ± 0.35 |
| Olipyphate (3;4), 100 mg/kg, 5 times daily, i.m. | 13.44 ± 2.12 | - |
| Olipyphate (3;4), 100 mg/kg, 5 times daily, per rectum, suppositories | 13.12 ± 1.60 | - |
| Olipyphate (3;4), 50 mg/kg, 5 times daily, per os | 20.90 ± 3.22 | - |
| Olipyphate (3;4), 100 mg/kg, 5 times daily, per os | 18.59 ± 2.82 | - |

**Table 2.**

| Cryogenic ulcer area in male and female rats under the action of Olipyphate | | | |
|---|---|---|---|
| Female rats | | Male rats | |
| Preparation | Ulcer area mm² | Preparation | Ulcer area, mm² |
| Cryogenic ulcer | 30.00 ± 6.68 | Cryogenic ulcer | 21.50 ± 2.99 |
| Olipyphate (1;2). 50 mg/kg, 5 times every second day, i.m. | 19.42 ± 3.87 | Olipyphate (1;2), 10 mg/kg, 5 times every second day, i.m. | 18.40 ± 2.73 |
| Olipyphate (1;2), 50 mg/kg, 3 times every second day, i.m. | 18.21 ± 3.27 | Olipyphate (1;2), 10 mg/kg, 3 times every second day, i.m. | 12.40 ± 2.93 |
| Olipyphate (3;4), 50 mg/kg, 5 times every second day, i.m. | 19.12 ± 3.23 | Olipyphate (3;4), 10 mg/kg, 5 times every second day, i.m. | 16.00 ± 2.45 |
| Olipyphate (3,4), 50 mg/kg, 3 times every second day, i.m. | 17.98 ± 3.09 | Olipyphate (3;4), 10 mg/kg, 3 times every second day, i.m. | 12.05 ± 2.66 |

### INDUSTRIAL USABILITY

The invention may be used in the production of the remedy for treating the wide range of ulcers.

## Claims

1. Use, in the manufacture of a medicament for the treatment of ulcers of a product obtainable by subjecting a lignin-containing raw material to process steps comprising:
basic hydrolysis-extraction;
oxidation with an oxygen-containing gas;
separation and acidification of the liquid phase; and
precipitation of the product.

2. Use according to claim 1 **characterized in that** hydrolysis-extraction is carried out in the field of ultrasonic oscillations.

3. Use according to claim 2 **characterized in that** hydrolysis-extraction is carried out under the conditions of the developed cavitation.

4. Use according to any of the claims 1 to 3 **characterized in that** a carrier is introduced into the medicament.

5. Use according to any of the claims 1-4 **characterized in that** the precipitated product is separated from the liquid phase, washed till neutral pH is reached, blended with pyrophosphate at a dry mass ratio of (25:17)±10% and dissolved in water while heating, NaCl is added to the solution in the amount of 3±10% mass parts and the desired product is obtained.

6. Use according to claim 5 **characterized in that** HCl is added to the desired product till neutral pH-value is reached.

## Patentansprüche

1. Verwendung, bei der Herstellung eines Medikamentes zur Behandlung von Ulcera, eines Produktes, erhaltbar durch Unterwerfen eines Lignin-enthaltenden Rohmaterials den Verfahrensschritten, die Folgendes umfassen:
basische Hydrolyse-Extraktion;
Oxidation mit einem Sauerstoff-enthaltenden Gas;
Trennung und Ansäuerung der Flüssigphase; und
Ausfällung des Produktes.

2. Verwendung nach Anspruch 1, **dadurch gekennzeichnet, dass** die Hydrolyse-Extraktion in dem Ultraschallschwingungsfeld durchgeführt wird.

3. Verwendung nach Anspruch 2, **dadurch gekennzeichnet, dass** die Hydrolyse-Extraktion unter den Bedingungen der entwickelten Kavitation durchgeführt wird.

4. Verwendung nach einem der Ansprüche 1 bis 3, **dadurch gekennzeichnet, dass** ein Träger in das Medikament eingeführt wird.

5. Verwendung nach einem der Ansprüche 1-4, **dadurch gekennzeichnet, dass** das ausgefällte Produkt von der Flüssigphase getrennt, bis zum Erreichen eines neutralen pH gewaschen, mit Pyrophosphat bei einem Trockenmasseverhältnis von (25:17) ± 10 % gemischt und während des Erhitzens in Wasser aufgelöst, der Lösung NaCl in der Menge von 3 ± 10 % Masseteilen zugefügt und das gewünschte Produkt erhalten wird.

6. Verwendung nach Anspruch 5, **dadurch gekennzeichnet, dass** dem gewünschten Produkt bis zum Erreichen eines neutralen pH-Wertes HCl zugefügt wird.

## Revendications

1. Utilisation, dans la fabrication d'un médicament pour le traitement d'ulcères, d'un produit pouvant être obtenu par la soumission d'une matière première contenant de la lignine aux étapes de procédé comprenant :
une hydrolyse alcaline-extraction ;
une oxydation par un gaz contenant de l'oxygène ;
une séparation et une acidification de la phase liquide ; et
une précipitation du produit.

2. Utilisation selon la revendication 1, **caractérisée en ce que** l'hydrolyse-extraction est réalisée dans le champ d'oscillations ultrasonores.

3. Utilisation selon la revendication 2, **caractérisée en ce que** l'hydrolyse-extraction est réalisée dans les conditions de la cavitation développée.

4. Utilisation selon l'une quelconque des revendications 1 à 3, **caractérisée en ce qu'**un véhicule est introduit dans le médicament.

5. Utilisation selon l'une quelconque des revendications 1 à 4, **caractérisée en ce que** le produit précipité est séparé de la phase liquide, lavé jusqu'à ce qu'un pH neutre soit atteint, mélangé avec du pyrophosphate à un rapport de masse sèche de (25:17) ± 10% et solubilisé dans l'eau tout en chauffant, on ajoute du NaCl à la solution en une quantité de 3 ± 10% parties en masse et le produit désiré est obtenu.

6. Utilisation selon la revendication 5, **caractérisée en ce que** l'on ajoute du HCl au produit désiré jusqu'à ce qu'une valeur de pH neutre soit atteinte.
